(19) Europäisches Patentamt
European Patent Office
Office européen des brevets

(11) Publication number: 0 068 239 A2

# (12) EUROPEAN PATENT APPLICATION

(21) Application number: 82105138.0

(22) Date of filing: 11.06.82

(51) Int. Cl.³: C 07 D 209/08, A 61 K 31/40

(30) Priority: 16.06.81 JP 91575/81

(43) Date of publication of application: 05.01.83 Bulletin 83/1

(84) Designated Contracting States: CH DE FR GB IT LI NL

(71) Applicant: MITSUI TOATSU CHEMICALS, INCORPORATED, 2-5 3-chome Kasumigaseki, Chiyoda-Ku Tokyo 100 (JP)

(72) Inventor: Yagami, Keisuke, 4-1350, Inage-cho, Chiba Chiba-ken (JP)
Inventor: Odate, Makoto, 1612, Kosugaya-sho Totsuka-ku, Yokohama Kanagawa-ken (JP)
Inventor: Yamashita, Hiroyuki, c/o Akio Funabashi 1-26-28 Hakusan, Bunkyo-ku, Tokyo (JP)
Inventor: Itoh, Toshihiko, 1-27-12, High Land, Yokosuka Kanagawa-ken (JP)
Inventor: Nishina, Takashi, 3-58, Hagiwara-cho, Mobara Chiba-ken (JP)
Inventor: Kamiya, Joji, 2141, Togo, Mobara Chiba-ken (JP)
Inventor: Awaya, Akira, 1541, Yabe-cho Totsuka-ku, Yokohama Kanagawa-ken (JP)
Inventor: Nakano, Takuo, 2231-20, Kamigo-cho Totsuka-ku, Yokohama Kanagawa-ken (JP)

(74) Representative: Schüler, Horst, Dr. European Patent Attorney, Kaiserstrasse 41, D-6000 Frankfurt/Main 1 (DE)

(54) **Sulfamoylbenzoic acid derivatives and pharmaceutical compositions comprising same.**

(57) Disclosed are sulfamoylbenzoic acid derivatives having the general formula

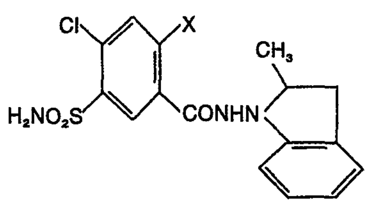

where X represents a chlorine or bromine atom. These compounds have antihypertensive activity, and pharmaceutical compositions for the treatment of hypertension comprising these compounds exert no diuretic effect.

EP 0 068 239 A2

# SPECIFICATION

Title of the Invention

Sulfamoylbenzoic Acid Derivatives and Pharmaceutical compositions Comprising Same

Background of the Invention

1. Field of the Invention

This invention relates to novel sulfamoylbenzoic acid derivatives having antihypertensive activity, a process for preparing the same, and pharmaceutical compositions comprising these compounds as the active ingredient.

2. Description of the Prior Art

Conventionally, it is well known that sulfamoylbenzoic acid derivatives have a diuretic effect, as is fully described in such publications as Journal of Medicinal Chemistry, Vol. 15, pp. 79-83, 1972. However, their diuretic effect may cause the development of a variety of undesirable symptoms such as hypokalemia, a reduction in glucose tolerance, uraturia, and the like. Accordingly, it is desirable to mitigate or eliminate such adverse effects.

Among the compounds somewhat similar in chemical structure to the compounds of the present invention is N-(3-sulfamoyl-4-chlorobenzamido)-2-methylindoline having the structural formula

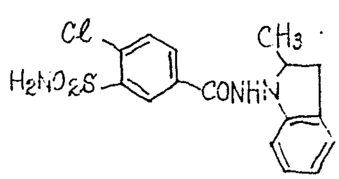

This compound is commonly known as a hypotensive and diuretic agent under the name of Indapamide (refer to, for example, Japanese Patent Publication No. 37818/'71). However, owing to its diuretic activity, adverse effects as described above may develop according to circumstances.

Summary of the Invention

It is an object of the present invention to provide a novel sulfamoylbenzoic acid derivative which, when used as the active ingredient of a pharmaceutical composition, exhibits powerful hypotensive activity without producing any adverse effect.

It is another object of the present invention to provide a pharmaceutical composition for the treatment of hypertension which exerts little or no diuretic effect.

The present inventors have made intensive studies in search of a compound which exhibits powerful hypotensive activity without producing any adverse effect, and have unexpectedly found that the novel compounds, or N-(2-halogeno-4-chloro-5-sulfamoylbenzamido)-2-methylindolines, obtained by introducing a chlorine or bromine atom into the 2-position of the benzene ring of the above-mentioned conventional compound N-(3-sulfamoyl-4-chlorobenzamido)-2-methylindoline exert little or no diuretic effect in spite of their powerful hypotensive activity.

In accordance with one feature of the present invention, there is provided a sulfamoylbenzoic acid derivative having the general formula

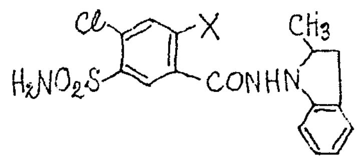

(I)

where X represents a chlorine or bromine atom.

In accordance with another feature of the present invention, there is provided a pharmaceutical composition comprising a sulfamoylbenzoic acid derivative of the general formula (I) and a pharmaceutical carrier.

Detailed Description of the Invention

The sulfamoylbenzoic acid derivatives within the scope of the general formula (I) can be synthesized, for example, according to the procedure illustrated by the following equations:

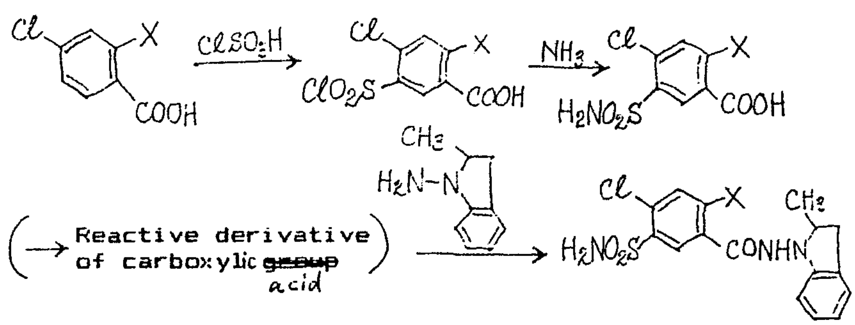


In the above equations, X has the same meaning as described above.

2-Halogeno-4-chloro-5-sulfamoylbenzoic acids can be prepared from 2-halogeno-4-chlorobenzoic acids, for example, according to the method described in Berichte, Vol. 99, p.328, 1966. The reaction of a 2-halogeno-4-chloro-5-sulfamoylbenzoic acid with

1-amino-2-methylindoline may be carried out by reacting the carboxylic acid and the amine in a solvent with the aid of cyclohexylcarbodiimide to condense them directly with elimination of water, by causing silicon tetrachloride to interact with the reactants in pyridine, or the like. Alternatively, a 2-halogeno-4-chloro-5-sulfamoylbenzoic acid may be converted to a reactive derivative thereof and then reacted with the amine. The reactive derivatives useful for this purpose include the acid chloride derived from the carboxylic acid, the mixed acid anhydride obtained by treating the carboxylic acid with ethyl chlorocarbonate, the active ester obtained by reacting the carboxylic acid with N-hydroxysuccinimide, and the like. The reaction of such a reactive derivative with the amine is carried out in a polar solvent such as tetrahydrofuran, pyridine, dimethylacetamide, dimethylurea, or the like. The reaction temperature may range from -20°C to the boiling point of the solvent, depending on the activity of the reactive derivative. In this reaction, triethylamine, dimethylaniline, and the like are used as condensation agents.

The pharmaceutical compositions of the present invention, which are useful in the treatment of hypertension, contain a sulfamoylbenzoic acid derivative of the general formula (I) in an amount of approximately 10 to 95% and preferably 15 to 90%. These pharmaceutical compositions may be prepared according to per se well-known techniques such as blending, granulation, sugar coating, dissolution, and lyophilization. By way of example, where they

are intended for oral use, the active ingredient is combined with a solid carrier and suitable pharmaceutic aids are added as desired. Specific examples of the useful carriers include sugars, cellulose preparations, calcium phosphate, and the like and specific examples of the useful pharmaceutic aids include binders, disintegrants (e.g., starch), flow controllers, lubricants, and the like. Moreover, any suitable additives may be incorporated according to the dosage form.

The present process for preparing sulfamoylbenzoic acid derivatives of the general formula (I) is illustrated by the following examples.

Example 1 [Synthesis of N-(2,4-dichloro-5-sulfamoylbenzamido)-2-methylindoline]

A mixture of 10.8 g of 2,4-dichloro-5-sulfamoylbenzoic acid and 40 ml of thionyl chloride was allowed to react at 80°C for 1 hour. After completion of the reaction, the formed 2,4-dichloro-5-sulfamoylbenzoyl chloride was separated by filtration and washed with benzene. A tetrahydrofuran solution containing 6 g of 1-amino-2-methylindoline and 4.1 g of triethylamine was kept at 5-10°C, and the acid chloride obtained as above was slowly added thereto. After completion of the reaction, the formed triethylamine hydrochloride was separated by filtration, and silica gel was added to the filtrate. Thus, the product was adsorbed on the silica gel and then purified by column chromatography to obtain a yield of 12.7 g of N-(2,4-dichloro-5-sulfamoylbenzamido)-2-methylindoline. When recrystallized from a mixture of water and ethanol, this product had a melting point of 218-220°C.

Nuclear magnetic resonance spectrum ($d_6$-DMSO, 100 MHz, TMS), ppm: 1.35 (3H, d), 2.4-2.7 (1H, m), 3.0-3.3 (1H, m), 3.6-4.1 (1H, m), 6.5-7.2 (4H, m), 7.6-7.8 (2H, s), 7.88 (1H, m), 8.0 (1H, m), 10.23 (1H, s).

Example 2 [Synthesis of N-(2-bromo-4-chloro-5-sulfamoylbenzamido)-2-methylindoline]

A mixture of 2 g of 2-bromo-4-chloro-5-sulfamoylbenzoic acid and 10 ml of thionyl chloride was heated under reflux for 1 hour. After this mixture was cooled, benzene was added thereto and the resulting precipitate was separated by filtration. The acid chloride thus obtained was added to a tetrahydrofuran solution containing 0.94 g of 1-amino-2-methylindoline and 0.89 ml of triethylamine. After completion of the reaction, the insoluble triethylamine hydrochloride was separated by filtration, and the filtrate was concentrated. Then, the product was crystallized by the addition of ether to obtain a yield of 2 g of N-(2-bromo-4-chloro-5-sulfamoylbenzamido)-2-methylindoline. When recrystallized from a mixture of water and ethanol, this product had a melting point of 240-242°C.

Nuclear magnetic resonance spectrum ($d_6$-DMSO, 100 MHz, TMS), ppm: 1.37 (3H, d), 2.5 (1H, m), 3.2 (1H, m), 3.9 (1H, m), 6.6-7.1 (4H, m), 7.8 (2H, s), 7.96 (1H, s), 8.04 (1H, s), 10.23 (1H, s).

The pharmacological activities of the sulfamoylbenzoic acid derivatives of the general formula (I) are demonstrated by the following evaluation tests.

Evaluation Test 1 [Hypotensive activity in rats with DOCA/saline-induced hypertension]

Rats with DOCA/saline-induced hypertension were prepared according to the method described in an article by H. Selye,

C.E. Hall and E.M. Rowly (Canadian Medical Association Journal, Vol. 49, p. 88, 1943). More specifically, 5-weeks-old male Wistar rats, weighing 100 to 120 g, were placed under anesthesia with ether and their left kidney was excised. Thereafter, they were allowed to take saline (a 1% aqueous solution of sodium chloride) instead of water. In addition, 10 mg/kg of deoxycorticosterone acetate was subcutaneously administered once a week. After 4-6 weeks, these rats developed hypertension.

Blood pressure was measured according to the method described in an article by M. Gerold and H. Tschirky (Arzneimittelforschung, Vol. 18, p. 1285, 1968). More specifically, using a Model 8002 or 8005 BP Recorder (W+W Electronic Co.), the systolic pressure of each rat was indirectly measured through the caudal artery. The rats were placed in a high-temperature environment (45±2°C) prior to every measurement. Among the rats (aged 15-17 weeks) with DOCA/saline-induced hypertension, only those showing a systolic pressure of 170 mmHg or above were used in groups of 3 or 4.

A compound to be tested was suspended in a 0.2% solution of carboxymethyl cellulose and administered orally to the rats in a dose of 30 mg/kg. This administration was performed once a day and continued for 5 days. Blood pressure measurements were made before the administration of the compound and 4 hour after the administration on days 1, 3, and 5.

The results thus obtained are summarized in Table 1.

Table 1

Percent Change of Systolic Pressure

| No. | Compound | Initial value (mmHg) | Percent change of systolic pressure | | |
|---|---|---|---|---|---|
| | | | Day 1 | Day 3 | Day 5 |
| 1 | Compound of Example 1 | 193 | -12 | -25 | -28 |
| 2 | Compound of Example 2 | 190 | -12 | -24 | -25 |
| 3 | Indapamide | 196 | -13 | -20 | -22 |

Evaluation Test 2 [Diuretic effect in anesthetized dogs]

Adult mongrel dogs of either sex were used in this experiment. Each dog was anesthetized by intravenous administration of 30 mg/kg of pentobarbital sodium. Thereafter, a midline incision was made in the lower abdominal region and polyethylene cannulae for the collection of urine were inserted into the right and left ureters. In addition, a polyethylene cannula was inserted into the left femoral vein, through which 50 ml of physiological saline was administered. Thirty minutes after that, physiological saline was continuously infused at a rate of 1.5 ml/min. The experiment was started after the rate of urine excretion became constant. A compound to be tested was dissolved in dimethylformamide and administered intravenously by way of the right femoral vein, and blood pressure was surgically monitored by way of the right femoral artery.

The $ED_{100}$ of the compound was defined as the dose at which the amount of urine collected for 10 minutes after the administration of the compound was equal to twice the amount of urine collected for 10 minutes before the administration. The results thus obtained are summarized in Table 2.

Table 2

| No. | Compound | $ED_{100}$ (mg/kg) |
|---|---|---|
| 1 | Compound of Example 1 | > 2 |
| 2 | Compound of Example 2 | > 2 |
| 3 | Indapamide | 0.2 |

Indapamide began to exert a diuretic effect at a dose of 0.05 mg/kg and increased urine excretion by a factor of approximately 2 at a dose of 0.2 mg/kg. In contrast, the compounds of Examples 1 and 2 exerted little diuretic effect even at a high dose of 2 mg/kg.

What is claimed is:

1. A sulfamoylbenzoic acid derivative having the general formula

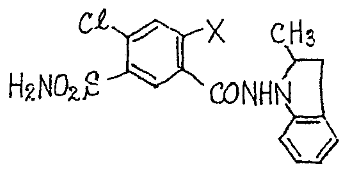

(I)

where X represents a chlorine or bromine atom.

2. A pharmaceutical composition for the treatment of hypertension comprising (a) a sulfamoylbenzoic acid derivative of the general formula

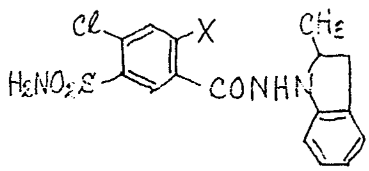

(I)

where X represents a chlorine or bromine atom, and (b) a pharmaceutical carrier.

3. A process for preparing sulfamoylbenzoic acid derivatives of the general formula

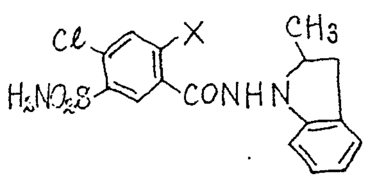

(I)

where X represents a chlorine or bromine atom, which comprises reacting a sulfamoylbenzoic acid of the general formula

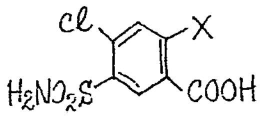

(II)

where X is as defined for the general formula (I), or a reactive derivative thereof with 1-amino-2-methylindoline.

4. The process according to claim 3 wherein the reactive derivative of sulfamoylbenzoic acid is the acid chloride derived therefrom.